# EUROPEAN PATENT SPECIFICATION

(11) **EP 2 917 346 B1**
(45) Date of publication and mention of the grant of the patent: **06.06.2018**
(21) Application number: 13811606.6
(22) Date of filing: 05.11.2013
(51) Int. Cl.: C12N 15/10

(54) **BACTERIAL ENGINEERING**
BAKTERIEN-ENGINEERING
INGÉNIERIE BACTÉRIENNE

(30) Priority: 06.11.2012 GB 201219989
(43) Date of publication of application: 16.09.2015
(73) Proprietor: Bactevo Ltd, Cambridge Cambridgeshire CB1 3LQ (GB)
(72) Inventor: WILLIAMS, David Hugh, Cambridge Cambridgeshire CB4 0WE (GB); TURNER, Arthur Keith, Cambridge Cambridgeshire CB4 0WE (GB); WAIN, John Richard, Cambridge Cambridgeshire CB4 0WE (GB)
(74) Representative: Hutchins, Michael Richard
(86) International application number: PCT/GB2013/052893
(87) International publication number: WO 2014/072697

(56) References cited:
- US-A1- 2009 104 682
- GEMMA C LANGRIDGE ET AL: "Simultaneous assay of every Salmonella Typhi gene using one million transposon mutants", GENOME RESEARCH, COLD SPRING HARBOR LABORATORY PRESS, WOODBURY, NY, US , vol. 19, no. 12 1 December 2009 (2009-12-01), pages 2308-2316, XP002682200, ISSN: 1088-9051, DOI: 10.1101/GR.097097.109 Retrieved from the Internet: URL:http://genome.cshlp.org/content/19/12/ 2308 [retrieved on 2009-10-13]
- MAUS C E ET AL: "Mutation of tlyA Confers Capreomycin Resistance in Mycobacterium tuberculosis", ANTIMICROBIAL AGENTS AND CHEMOTHERAPY, AMERICAN SOCIETY FOR MICROBIOLOGY, US, vol. 49, no. 2, 1 February 2005 (2005-02-01), pages 571-577, XP007901245, ISSN: 0066-4804, DOI: 10.1128/AAC.49.2.571-577.2005
- JUN LIN ET AL: "Systematic Identification of Genetic Loci Required for Polymyxin Resistance in Campylobacter jejuni Using an Efficient In Vivo Transposon Mutagenesis System", FOODBORNE PATHOGENS AND DISEASE, MARY ANN LIEBERT, INC. PUBLISHERS, US, vol. 6, no. 2, 1 March 2009 (2009-03-01), pages 173-185, XP002682201, ISSN: 1535-3141, DOI: 10.1089/FPD.2008.0177 [retrieved on 2008-12-23]
- HARE R S ET AL: "Genetic footprinting in bacteria", JOURNAL OF BACTERIOLOGY, AMERICAN SOCIETY FOR MICROBIOLOGY, WASHINGTON, DC; US, vol. 183, no. 5, 1 March 2001 (2001-03-01) , pages 1694-1706, XP002243541, ISSN: 0021-9193, DOI: 10.1128/JB.183.5.1694-1706.2001
- TONG X ET AL: "Genome-scale identification of conditionally essential genes in E. coli by DNA microarrays", BIOCHEMICAL AND BIOPHYSICAL RESEARCH COMMUNICATIONS, ACADEMIC PRESS INC. ORLANDO, FL, US, vol. 322, no. 1, 10 September 2004 (2004-09-10), pages 347-354, XP004526740, ISSN: 0006-291X, DOI: 10.1016/J.BBRC.2004.07.110
- JIAO YONGQIN ET AL: "Isolation and characterization of a genetically tractable photo autotrophic Fe(II)-oxidizing bacterium, Rhodopseudomonas palustris strain TIE-1", APPLIED AND ENVIRONMENTAL MICROBIOLOGY, vol. 71, no. 8, August 2005 (2005-08), pages 4487-4496, XP002720185, ISSN: 0099-2240
- PUTRINS MARTA ET AL: "The impact of ColRS two-component system and TtgABC efflux pump on phenol tolerance of Pseudomonas putida becomes evident only in growing bacteria", April 2010 (2010-04), BMC MICROBIOLOGY, VOL. 10, PAGE(S) ARTICLE NO.: 110, XP002720186, ISSN: 1471-2180 Abstract; page 2, column 2, last paragraph - page 3, column 1, paragraph 1
- KOMATSU MAMORU ET AL: "Genome-minimized Streptomyces host for the heterologous expression of secondary metabolism.", 9 February 2010 (2010-02-09), PROCEEDINGS OF THE NATIONAL ACADEMY OF SCIENCES OF THE UNITED STATES OF AMERICA 9 FEB 2010, VOL. 107, NR. 6, PAGE(S) 2646 - 2651, XP002720187, ISSN: 1091-6490 the whole document
- CIAMPI M S ET AL: "TRANSPOSON TN-10 PROVIDES A PROMOTER FOR TRANSCRIPTION OF ADJACENT SEQUENCES", PROCEEDINGS OF THE NATIONAL ACADEMY OF SCIENCES, NATIONAL ACADEMY OF SCIENCES, US, vol. 79, no. 16, 1 August 1982 (1982-08-01), pages 5016-5020, XP002682203, ISSN: 0027-8424
- S. J. Salipante ET AL: "GeneHunter, a Transposon Tool for Identification and Isolation of Cryptic Antibiotic Resistance Genes", ANTIMICROBIAL AGENTS AND CHEMOTHERAPY, vol. 47, no. 12, 1 December 2003 (2003-12-01), pages 3840-3845, XP055270336, ISSN: 0066-4804, DOI: 10.1128/AAC.47.12.3840-3845.2003

## Description

### Field of the Invention

The present disclosure relates to processes for engineering bacterial cells for use in biotechnological applications, including the production of proteins, secondary metabolites and biofuels, biocatalysis, bioremediation, biotransformation, biodegradation, biological control, drug development, drug screening, vaccines, probiotics, biosensors and drug delivery vehicles.

### Background to the Invention

Bacteria find application in many aspects of biotechnology, including use as hosts for the production of heterologous proteins and peptides (including enzymes and therapeutic antibodies), as the source of secondary metabolites or their derivatives, as agents for biological control, biodegradation, biofuel production, biocatalysis and bioremediation and as probiotics, vaccine components and drug delivery systems.

Naturally occurring bacterial strains are not optimized for biotechnological use. One of the aims of the emerging field of synthetic biology is the engineering of new organisms which are more tractable and/or more efficient as biotechnological tools.

One approach may be termed the "ground-up" approach (see e.g. WO2008/024129). This involves the synthesis of a minimalized, artificial bacterial DNA genome containing only those genes essential for growth. This is then used to create a bare "chassis" to which desired biosynthetic pathways, signalling pathways or catabolic functions can be added as required. This approach is currently impractical, since gene products and regulatory elements synergize and cross-talk in the context of the whole cell in ways which are currently incompletely understood and which cannot therefore be treated as formally modular.

Another approach is termed the "strip down" approach. This currently finds application in the production of useful secondary metabolites in *Streptomyces* spp. Here, genes and other genetic material not essential for growth are removed ("stripped out"), and those for selected biosynthetic pathways reintroduced individually. For example, Komatsu et al. (2010) PNAS 107 (6): 2646-2651 describe the construction of an engineered bacterium for heterologous expression of genes encoding secondary metabolite biosynthesis involving systematic deletion of nonessential genes from the genome of the industrial microorganism *Streptomyces avermitilis.*

The "strip down" approach requires methods for identifying genes, which are inessential (and so metabolically costly and therefore disadvantageous) for survival and/or growth under selected conditions.

Moreover, the approach would benefit from complementary functional genomic analyses to also identify genes which are advantageous for the proposed biotechnological applications. In the latter case, a "strip down/rev-up" approach could then be employed to minimize the metabolic burden arising from non-essential (disadvantageous) genes, while amplifying the beneficial effect of genes which directly or indirectly contribute to the biotechnological application (i.e. advantageous genes).

Transposon directed insertion-site sequencing (TraDIS - see Langridge et al. (2009) Genome Research 19: 2308-2316) has recently been described and used to identify: (a) essential genes; (b) genes advantageous (but not essential) for growth; (c) genes disadvantageous for growth under particular conditions; and (d) genes involved in conferring tolerance to certain conditions ("niche-specific" essential genes). Similar techniques have been described in e.g. Gawronski et al. (2009) PNAS 106: 16422-16427; Goodman et al. (2009) Cell Host Microbe 6: 279-289; van Opijnen et al. (2009) Nat. Methods 6: 767-772 and Gallagher et al. (2011) mBio 2(1):e00315-10, and such techniques are now collectively dubbed "Tn-seq" methods.

The present inventors have now discovered that TraDIS can be adapted to provide an extremely elegant solution to the problem of unequivocally identifying both disadvantageous and advantageous genes for the purposes of bacterial bioengineering. This is achieved *via* the use of activating transposons (Tn_{A}). These transposons comprise a promoter such that transposon insertion into bacterial DNA at a suitable insertion site increases the transcription of a gene at or near that insertion site. Mutagenesis with Tn_{A} therefore yields insertionally-inactivated mutants (in which the Tn_{A} has disrupted gene expression, typically after insertion into the coding region) as well as insertionally-activated mutants (typically where the transposon has inserted upstream of a gene such that the promoter drives high level transcription (and consequently produces higher levels of expression) of the gene.

### Summary of the Invention

According to the present disclosure there is provided a process for producing a mutant bacterium which exhibits improved survival and/or growth under a selected growth condition, the process comprising the steps of:
a) generating a pool of mutant bacteria by transposon mutagenesis with an activating transposon (Tn_{A}), wherein the Tn_{A} comprises a promoter capable of increasing transcription of a gene at or near its insertion site;
b) growing bacteria from the mutant pool under the selected growth condition and under one or more reference conditions to produce two or more test cultures; and
c) comparing the distribution of Tn_{A} insertions between test cultures to identify a first class of genes which are disadvantageous for growth and/or survival under the selected growth condition and a second class of genes which are advantageous for growth and/or survival under the selected growth condition.

Typically, Tn_{A} insertions associated with the first class of (disadvantageous) genes occur in the coding region (so that the gene is insertionally inactivated by the Tn_{A}) or outside of the coding region, but on the complimentary DNA strand to the coding sequence (causing either generation of antisense RNA or promoter activity disruption), whereas Tn_{A} insertions associated with the second (advantageous) class of genes occur upstream of the coding region in an orientation whereby the promoter of the Tn_{A} drives elevated transcription (and so expression) of the gene.

The process may further comprise the step of providing an engineered mutant bacterium in which at least one of said disadvantageous genes is removed or disrupted and/or at least one of said advantageous gene is overexpressed, such that the mutant bacterium exhibits improved survival and/or growth under the selected growth condition. In such embodiments, a plurality of said disadvantageous genes may be removed or disrupted while a plurality of advantageous genes is overexpressed.

In this way, a mutant bacterium which is significantly better adapted to the selected growth condition (and so to the proposed biotechnological use) may be engineered or selected.

In such embodiments, the process may further comprise isolating and culturing the engineered mutant bacterium and then subjecting it to a further round of mutagenesis, culture and comparison (as defined in steps (a)-(c), above), and may optionally further comprise the step of providing a second round engineered mutant bacterium in which at least one of said further disadvantageous genes is removed or disrupted and/or at least one of said further advantageous gene is overexpressed, such that the mutant bacterium exhibits further improved survival and/or growth under the selected growth condition relative to the engineered mutant bacterium produced after the first round of mutagenesis. Thus, the process of the invention is preferably iterative, and may comprise yet further rounds of mutagenesis and iterative application of steps (a) to (c) (as defined above) to provide a third, fourth, fifth (or greater) round mutant bacterium which exhibits yet further improved survival and/or growth in the presence of said environmental challenge relative to the engineered mutant bacterium of the preceding round.

In such embodiments, the selected growth condition applied during each round may be the same or different.

Other aspects and preferred embodiments of the invention are defined and described in the other claims set out below.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The term *gene* is a term describing a hereditary unit consisting of a sequence of DNA that occupies a specific location on a chromosome or plasmid and determines a particular characteristic, or group of characteristics, in an organism. A gene may determine a characteristic of an organism by specifying a polypeptide chain that forms a protein or part of a protein (structural gene); or encode an RNA molecule; or by specifying nucleic acid that forms a structural entity that influences, or in any way, regulates the operation of other genes or repress such operation (e.g. by acting in *cis*); or affect phenotype by some other as yet undefined mechanism.

The terms *genomic DNA* is a term of art used herein to define chromosomal DNA as distinct from extrachromosomally-maintained plasmid DNA.

The term *genome* is a term of art used herein to define the entire genetic complement of an organism, and so includes chromosomal, plasmid, prophage and any other DNA or RNA acting a the genetic material..

The term *Gram-positive bacterium* is a term of art defining a particular class of bacteria that are grouped together on the basis of certain cell wall staining characteristics.

The term *low G+C Gram-positive bacterium* is a term of art defining a particular subclass class of evolutionarily related bacteria within the Gram-positives on the basis of the composition of the bases in the DNA. The subclass includes *Streptococcus* spp., *Staphylococcus* spp., *Listeria* spp., *Bacillus* spp., *Clostridium* spp., *Enterococcus* spp. and *Lactobacillus* spp.).

The term *high G+C Gram-positive bacterium* is a term of art defining a particular subclass class of evolutionarily related bacteria within the Gram-positives on the basis of the composition of the bases in the DNA. The subclass includes actinomycetes (actinobacteria) including *Actinomyces* spp., *Arthrobacter* spp., *Corynebacterium* spp., *Frankia* spp., *Micrococcus* spp., *Micromonospora* spp., *Mycobacterium* spp., *Nocardia* spp., *Propionibacterium* spp. and *Streptomyces* spp.

The term *Gram-negative bacterium* is a term of art defining a particular class of bacteria that are grouped together on the basis of certain cell wall staining characteristics. Examples of Gram-negative bacterial genera include *Klebsiella, Acinetobacter, Escherichia, Pseudomonas, Enterobacter* and *Neisseria.*

### Selection of growth conditions

The processes of the invention permit the engineering of mutant bacteria which exhibit improved survival and/or growth under selected growth conditions, and involve detecting differences in the distribution and/or frequency of Tn_{A} insertions under a selected growth condition relative to one or more reference conditions.

The growth condition is selected according to the desired biotechnological application of the engineered bacteria ultimately produced: Any condition is suitable providing that differences between the selected and reference growth conditions drive a shift in the distribution of recoverable Tn_{A} insertion mutants in a test culture derived from the initial pool of Tn_{A} insertion mutants.

In certain embodiments, the selected growth condition is characterized by the presence of one or more selective agents which are absent (or present at a different, e.g. lower or higher, concentration in the reference condition(s)). In this context, the term "selective agent" is used in a broad sense to cover any agent (or combination of agents) which causes a change in the distribution and/or frequency of genomic Tn_{A} insertions when used in the process of the invention.

Such agents therefore include without limitation: environmental pollutants; toxins; antibiotics; carbon sources; nitrogen sources; energy sources; other microbes (e.g. yeasts, viruses, bacteria and/or plants); pH; pressure; temperature; salt concentration; pesticides; radioactive material; hydrocarbons; oil residues; industrial waste products; medical waste products; wastewater residues and the like.

Exemplary, non-limiting selective agents for use in providing the selected growth conditions of the processes of the invention according to the intended biotechnological use of the engineered bacteria are listed below:
*Bioremediation* - arsenic, metals, for example heavy metals and in particular lead, mercury uranium, palladium, chromium and cadmium; polynuclear aromatic hydrocarbons, chlorinated solvents, phenols, oils, pesticides and phosphates.
*Microbial enhanced oil recovery* - Crude oil and heavy oil fractions
*Sewage treatment* - nitrites, ammonia, phosphates and oestrogen-like compounds.
*Food production* - Lactose and acetic acid.
*Biofuel production* - Carbon dioxide, hydrogen, sunlight, oxygen, cellulose and hemicellulose.
*Energy Generation* - Waste water, marine sediment, freshwater sediment, river water, acetate, propionate and butyrate.
Bio-production and vaccines - Luria-Bertani or LB broth, Terrific Broth (TB), 2YT or chemically defined media.
*Bio-digestion*/*biodegradation* - Straw fractions, cellulosic waste and plastics.
*Probiotics* - Other bacteria, for example members of the Firmicutes phylum.
*Treatment of infection* - Pathogenic bacteria, plants, *Agrobacterium tumefaciens,* animals, *Staphylococcus aureus,* human tissue and *Clostridium difficile,* human commensal bacteria or human mutualistic bacteria.

### Bacteria for use in the methods of the invention

The processes of the invention may be applied to the engineering of any bacterium. The bacterium selected will depend *inter alia* on the intended biotechnological use.

Thus, the processes of the invention find application in the identification of antibiotic targets in: (a) Gram-positive, Gram-negative and/or Gram-variable bacteria; (b) spore-forming bacteria; (c) non-spore forming bacteria; (d) filamentous bacteria; (e) intracellular bacteria; (f) obligate aerobes; (g) obligate anaerobes; (h) facultative anaerobes; (i) microaerophilic bacteria and/or (f) opportunistic bacterial pathogens.

In certain embodiments, the methods of the invention are applied to bacteria of the following genera: *Acinetobacter (e.g. A. baumannii); Aeromonas* (e.g. A. *hydrophila); Bacillus* (e.g. *B. anthracis); Bacteroides* (e.g. *B. fragilis); Bordetella* (e.g. *B. pertussis*); *Borrelia* (e.g. *B. burgdorferi*); *Brucella* (e.g. *B. abortus, B. canis, B. melitensis* and *B. suis*); *Burkholderia* (e.g. *B. cepacia* complex); *Campylobacter* (e.g. *C. jejum*); *Chlamydia* (e.g. *C*. *trachomatis, C. suis and C. muridarum*); *Chlamydophila* (e.g. (e.g. *C. pneumoniae, C. pecorum, C. psittaci, C. abortus, C. felis and C. caviae*); *Citrobacter* (e.g. *C. freundii*); *Clostridium* (e.g. *C. botulinum, C. difficile, C. perfringens* and *C. tetam*); *Corynebacterium* (e.g. *C. diphteriae* and *C. glutamicum*); *Enterobacter* (e.g. *E. cloacae* and *E. aerogenes*); *Enterococcus* (e.g. *E. faecalis* and *E. faecium*); *Escherichia* (e.g. *E. coli*); *Flavobacterium; Francisella* (e.g. *F. tularensis*); *Fusobacterium* (e.g. *F. necrophorum*); *Haemophilus* (e.g. *H. somnus, H. influenzae* and *H. parainfluenzae*); *Helicobacter* (e.g. *H. pylori*); *Klebsiella* (e.g. *K. oxytoca* and *K. pneumoniae*), *Legionella* (e.g. *L. pneumophila*); Leptospira (e.g. *L*. *interrogans*); *Listeria* (e.g. *L. monocytogenes*); *Moraxella* (e.g. *M. catarrhalis*); *Morganella* (e.g. *M. morganii*); *Mycobacterium* (e.g. *M. leprae* and *M. tuberculosis*); *Mycoplasma* (e.g. *M. pneumoniae*); *Neisseria* (e.g. *N. gonorrhoeae* and *N. meningitidis*); *Pasteurella* (e.g. *P*. *multocida*); *Peptostreptococcus; Prevotella; Proteus* (e.g. *P. mirabilis* and *P. vulgaris*), *Pseudomonas* (e.g. *P. aeruginosa*); *Rickettsia* (e.g. *R. rickettsii*); *Salmonella* (e.g. serotypes . Typhi and Typhimurium); *Serratia* (e.g. *S. marcesens*); *Shigella* (e.g. *S. flexnaria, S. dysenteriae* and *S. sonnei*); *Staphylococcus* (e.g. *S. aureus, S. haemolyticus, S. intermedius, S. epidermidis* and *S. saprophyticus*); *Stenotrophomonas* (e.g. *S. maltophila*); *Streptococcus* (e.g. *S. agalactiae, S. mutans, S. pneumoniae* and *S. pyogenes*); *Treponema* (e.g. *T. pallidum*); *Vibrio* (e.g. *V. cholerae*) and *Yersinia* (e.g. *Y. pestis*).

Exemplary, non-limiting bacteria for use in the processes of the invention according to the intended biotechnological use are listed below:
*Bioremediation* - *Acinetobacter, Pseudomonas, Alcaligenes, Sphingomonas, Rhodococcus, Mycobacterium, Geobacter, Cupriavidus* and *Desulfovibrio.*
*Microbial enhanced oil recovery* - *Acinetobacter, Bacillus, Pseudomonas, Rhodococcus, Arthrobacter, Klebsiella* and *Clostridium.*
*Sewage treatment* - *Acinetobacter, Nitrobacter, Nitrococcus and Nitrospira.*
*Food production* - *Acetobacter.*
*Biofuel production* - *Ralstonia eutropha, Halanaerobium hydrogeniformans, Escherichia coli,* Cyanobacteria, *Clostridium acetobutylicum, Zymomonas mobilis* and *Caldicellulosiruptor obsidiansis.*
*Energy generation* - *Geobacter, Desulfuromonas, Proteobacterium, Pelobacter Thauera, Bacillus* and *Dechloromonas*
*Bio-production* - Escherichia coli, *Bacillus brevis, Bacillus megaterium, Bacillus subtilis, Caulobacter crescentus,* Streptomyces,
*Bio-digestion*/*biodegradation*
*Ralstonia eutropha, Halanaerobium hydrogeniformans, Escherichia coli, Cyanobacteria, Clostridium acetobutylicum, Zymomonas mobilis, Caldicellulosiruptor obsidiansis, Vaccines*
*Caulobacter crescentus, Escherichia coli,* Salmonella
*Probiotics* - *Bifidobacterium,* members of the Firmicutes phylum, human commensal bacteria and human mutualistic bacteria.
*Treatment of Infection* - competing "good" bacteria (e.g. human commensal bacteria or human mutualistic bacteria), plants, avirulent strains of *Agrobacterium, Staphylococcus hominis and Bifidobacterium.*

### Genomic engineering

The process of the invention may comprise the step of providing an engineered mutant bacterium in which at least one of said disadvantageous genes is removed or disrupted and/or at least one of said advantageous gene is overexpressed, such that the mutant bacterium exhibits improved survival and/or growth under the selected growth condition. With regard to the removal or disruption of disadvantageous/inessential genes, various experimental procedures for chromosomal gene deletion/replacement in bacteria, which enable the specific substitution of targeted genome sequences with copies of those carrying defined mutations, are known in the art.

Two methods are of particular utility: the first ("in-out") method is based on integration of plasmid DNA into the bacterial chromosome and subsequent resolution of the co-integrate. The second ("linear fragment" or recombineering) method is based on homologous recombination mediated by short homology arms at the ends of linear DNA molecule.

These methods are reviewed in e.g. Madyagol et al. (2011) Folia Microbiol 56: 253-263. Such methods may be used to delete large tracts of the bacterial genome, and if used to eliminate all (or substantially all) genes inessential for the desired biotechnological application (i.e. all dispensable genes that would otherwise impose a metabolic burden upon the bacterial cell), the process may be termed "genome minimization".

In some embodiments, the mutant bacterium may be engineered to have a "minimized" genome that is smaller by 10%, 20%, 30%, 40%, 50%, 60%, 70%, 80%, 90% or more than 90% as compared to the wild type genome.

With regard to the overexpression of advantageous genes such that the mutant bacterium exhibits improved survival and/or growth under the selected growth condition, any of a wide variety of known techniques may be used, including *inter alia* the introduction of chemically-induced (or u.v. induced) point mutations, insertion of strong promoters, ribosome binding sites, removal of repressor binding sites and optimization of codon usage.

### Mutant pools

The methods of the invention involve generating a pool of mutant bacteria by transposon mutagenesis. The size of the mutant pool affects the resolution of the method: as the pool size increases, more and more different genes with Tn_{A} insertions will be represented (and so effectively assayed). As the pool size decreases, the resolution of the method reduces, genes will be less effectively assayed, and more and more genes will not be assayed at all.

Ideally, the mutant pool generated in the methods of the invention is *comprehensive,* in the sense that insertions into every gene are represented. The number of Tn_{A} insertion mutants (i.e. the mutant pool size) required to achieve this depends on various factors, including: (a) the size of the bacterial genome; (b) the average size of the genes; and (c) any Tn_{A} insertion site bias.

With regard to the latter, some areas of bacterial genomes attract a low frequency of insertion (especially GC-rich regions). Thus, insertion frequencies and pool sizes large enough to ensure that insertions into insertion-refractory regions are preferred.

In general, a minimum insertion rate of one transposon per 25bp is required to achieve a comprehensive pool/library, which typically entails a minimum pool size for bacteria having a genome size of 4 to 7 Mb of 0.5 x 10⁵ to 1 x 10⁵, for example 5x10⁵, preferably at least about 1x10⁶ mutants. In many cases, 1x10⁶ mutants will allow identification of ∼300,000 different insertion sites and correspond to 1 transposon insertion every 13 to 23 bp (or about 40-70 different insertion sites per gene).

However, the methods of the invention do not necessarily require a comprehensive mutant pool (in the sense defined above) in order to return useful information. Rather, pool sizes less than the ideal comprehensive pool may be used, provided that a reduction in resolution (and attendant failure to assay certain genes) can be tolerated. This may be the case, for example, where the method is designed to be run iteratively until the target is identified: in such embodiments the effective pool size grows with each iteration of the method.

### Transposon mutagenesis

Transposons, sometimes called transposable elements, are polynucleotides capable of inserting copies of themselves into other polynucleotides. The term transposon is well known to those skilled in the art and includes classes of transposons that can be distinguished on the basis of sequence organisation, for example short inverted repeats at each end; directly repeated long terminal repeats (LTRs) at the ends; and polyA at 3'ends of RNA transcripts with 5' ends often truncated.

Transposomes are transposase-transposon complexes wherein the transposon does not encode transposase activity. Thus, once inserted the transposon is stable. Preferably, in order to ensure mutant pool stability, the transposon does not encode transposase and is provided in the form of a transposome (i.e. as a complex with transposase enzyme), as described below.

As used herein, the term "activating transposon" (hereinafter abbreviated "Tn_{A}") defines a transposon which comprises a promoter such that transposon insertion increases the transcription of a gene at or near the insertion site. Examples of such transposons are described in Troeschel et al. (2010) Methods Mol Biol. 668:117-39 and Kim et al. (2008) Curr Microbiol. 57(4): 391-394.

The activating transposon/transposome can be introduced into the bacterial genome (including chromosomal and/or plasmid DNA) by any of a wide variety of standard procedures which are well-known to those skilled in the art. For example, Tn_{A} transposomes can be introduced by electroporation (or any other suitable transformation method).

Preferably, the transformation method generates 1x10³ to 5x10³ transformants/ng DNA, and such transformation efficiencies are generally achievable using electroporation.

Alternatively, transposon mutagenesis using Tn_{A} may be performed *in vitro* and recombinant molecules transformed/transfected into bacterial cells. In such embodiments, transposomes can be prepared according to a standard protocol by mixing commercially available transposase enzyme with the transposon DNA fragment. The resulting transposomes are then mixed with plasmid DNA of the plasmid of interest to allow transposition, then the DNA introduced into a host bacterial strain using electrotransformation to generate a pool of plasmid transposon mutants.

In embodiments where mutagenesis is performed *in vitro,* it is possible to mix transposomes with genomic DNA *in vitro* and then introduce the mutagenized DNA (optionally, after fragmentation and/or circularization) into the host bacterial strain (e.g. by electroporation) whereupon endogenous recombination machinery incorporates it into the genome. Such an approach may be particularly useful in the case of bacteria which are naturally competent (e.g. *Acinetobacter* spp.) and/or can incorporate DNA *via* homologous crossover (e.g. double crossover) recombination events.

### Activating transposons for use in the methods of the invention

Any suitable activating transposon may be used in the methods of the invention. Suitable transposons include those based on Tn*3* and the Tn*3*-like (Class II) transposons including *γδ* (Tn *1000*), Tn*501*, Tn*2501*, Tn*21*, Tn*917* and their relatives. Also Tn*10*, Tn*5*, Tn*pho*A, Tn*903*, bacteriophage Mu and related transposable bacteriophages. A variety of suitable transposons are also available commercially, including for example the EZ-Tn5™ <R6Kγori/KAN-2> transposon.

Preferred transposons are those which carry antibiotic resistance genes (which may be useful in identifying mutants which carry a transposon) including Tn*5*, Tn*10* and Tn*pho*A. For example, Tn*10* carries a tetracycline resistance gene between its IS elements while Tn*5* carries genes encoding polypeptides conferring resistance to kanamycin, streptomycin and bleomycin. Other suitable resistance genes include those including chloramphenicol acetyltransferase (conferring resistance to chloramphenicol).

It is of course possible to generate new transposons by inserting different combinations of antibiotic resistance genes between IS elements, or by inserting combinations of antibiotic resistance genes between transposon mosaic ends (preferred), or by altering the polynucleotide sequence of the transposon, for example by making a redundant base substitution or any other type of base substitution that does not affect the transposition or the antibiotic resistance characteristics of the transposon, in the coding region of an antibiotic resistance gene or elsewhere in the transposon. Such transposons are included within the scope of the invention.

In many embodiments, a single transposon is used to generate the mutant pool. However, as explained above, the number of Tn insertion mutants (i.e. the mutant pool size) required to achieve a comprehensive pool or library depends *inter alia* on any Tn insertion site bias. Thus, in cases where the transposon insertion site bias occurs, two or more different transposons may be used in order to reduce or eliminate insertion site bias. For example, a combination of two different transposons based on Tn*5* and Tn*10* may be employed.

### Promoters for use in activating transposons

The nature of the promoter present in the Tn_{A} is dependent on the nature of the transposon and the ultimate bacterial host. Generally, an efficient, outward-oriented promoter which drives high level transcription of DNA near or adjacent to the insertion site is chosen.

The promoter may include: (a) a Pribnow box (-10 element); (b) a-35 element and/or (c) an UP element.

For example, the *lac* promoter can be used with the EZ-Tn5™ <R6Kγori/KAN-2> transposon, and such constructs are suitable for assay of e.g. *Escherichia coli, Enterobacter* spp. and other members of the family *Enterobacteriaceae* such as *Klebsiella* spp. Other suitable promoters include: *rpl*J (large ribosomal subunit protein; moderate strength promoter); *tac* (artificial *lac*/*trp* hybrid; strong promoter) and *rrnB* (ribosomal RNA gene promoter; very strong promoter).

### Determining the distribution of Tn_{A} insertions

The distribution of transposon insertions is preferably determined by sequencing bacterial DNA adjacent or near (5' and/or 3') the Tn_{A} insertion site (e.g. by sequencing DNA which comprises Tn_{A} -genomic DNA junctions). Typically, bacterial DNA flanking or adjacent to one or both ends of the Tn_{A} is sequenced.

The length of adjacent DNA sequenced need not be extensive, and is preferably relatively short (for example, less than 200 base pairs).

Various methods can be used to determine the Tn_{A} insertion distribution using DNA sequencing: such methods have recently been dubbed Tn-seq procedures (van Opijnen et al. (2009) Nat. Methods 6: 767-772). For example, Tn-seq procedures include affinity purification of amplified Tn junctions (Gawronski et al. (2009) PNAS 106: 16422-16427); ligation of adaptors into genome sequences distal to the end of the transposon using a specialized restriction site (Goodman et al. (2009) Cell Host Microbe 6: 279-289; van Opijnen et al. (2009) Nat. Methods 6: 767-772); selective amplification (Langridge et al. (2009) Genome Research 19: 2308-2316) and the generation of single-stranded DNA circles bearing Tn junctions, which serve as templates for amplification and sequencing after elimination of genomic DNA by exonuclease digestion (Gallagher et al. (2011) mBio 2(1):e00315-10).

Any suitable high-throughput sequencing technique can be used, and there are many commercially available sequencing platforms that are suitable for use in the methods of the invention. Sequencing-by-synthesis (SBS)-based sequencing platforms are particularly suitable for use in the methods of the invention: for example, the Illumina™ system generates millions of relatively short sequence reads (54, 75 or 100bp) and is particularly preferred.

Other suitable techniques include methods based on reversible dye-terminators. Here, DNA molecules are first attached to primers on a slide and amplified so that local clonal colonies are formed (bridge amplification). Four types of ddNTPs are added, and non-incorporated nucleotides are washed away. Unlike pyrosequencing, the DNA can only be extended one nucleotide at a time. A camera takes images of the fluorescently labeled nucleotides then the dye along with the terminal 3' blocker is chemically removed from the DNA, allowing a next cycle.

Other systems capable of short sequence reads include SOLiD™ and Ion Torrent technologies (both sold by Applied Biosystems™). SOLiD™ technology employs sequencing by ligation. Here, a pool of all possible oligonucleotides of a fixed length are labeled according to the sequenced position. Oligonucleotides are annealed and ligated; the preferential ligation by DNA ligase for matching sequences results in a signal informative of the nucleotide at that position. Before sequencing, the DNA is amplified by emulsion PCR. The resulting bead, each containing only copies of the same DNA molecule, are deposited on a glass slide. The result is sequences of quantities and lengths comparable to Illumina sequencing.

Ion Torrent Systems Inc. have developed a system based on using standard sequencing chemistry, but with a novel, semiconductor based detection system. This method of sequencing is based on the detection of hydrogen ions that are released during the polymerisation of DNA, as opposed to the optical methods used in other sequencing systems. A microwell containing a template DNA strand to be sequenced is flooded with a single type of nucleotide. If the introduced nucleotide is complementary to the leading template nucleotide it is incorporated into the growing complementary strand. This causes the release of a hydrogen ion that triggers a hypersensitive ion sensor, which indicates that a reaction has occurred. If homopolymer repeats are present in the template sequence multiple nucleotides will be incorporated in a single cycle. This leads to a corresponding number of released hydrogens and a proportionally higher electronic signal.

### Functional assessment of genes

The genes identified by comparing the distribution of Tn_{A} insertions between test cultures may be further characterized by various techniques which directly or indirectly assess its function. In this way, an essential function may be definitively assigned to said gene.

Suitable techniques include bioinformatics, where the (full or partial) sequence of the putative essential gene is used to interrogate sequence databases containing information from the bacterium assayed and/or other species in order to identify genes (e.g. orthologous genes in other species) for which essential biochemical function(s) have already been assigned and/or which have been shown to be essential.

Suitable bioinformatics programs are well known to those skilled in the art and include the Basic Local Alignment Search Tool (BLAST) program (Altschul et al. (1990) J. Mol. Biol. 215: 403-410 and Altschul et al. (1997) Nucl. Acids Res. 25: 3389-3402). Suitable databases include, for example, EMBL, GENBANK, TIGR, EBI, SWISS-PROT and trEMBL.

Alternatively, or in addition, the (full or partial) sequence of the gene is used to interrogate a sequence database containing information as to the identity of essential genes which has been previously constructed using the conventional Tn-seq methods described in the prior art (e.g. as described in Gawronski et al. (2009) PNAS 106: 16422-16427; Goodman et al. (2009) Cell Host Microbe 6: 279-289; van Opijnen et al. (2009) Nat. Methods 6: 767-772; Langridge et al. (2009) Genome Research 19: 2308-2316; Gallagher et al. (2011) mBio 2(1):e00315-10) and/or the techniques described in WO 01/07651. Despite the presence of a promoter within the inserted sequence, many Tn_{A} insertions will disrupt gene/DNA function and allow identification of essential/important DNA regions, as in standard Tn-seq (including TraDIS). However, some transposons will be positioned appropriately with respect to specific DNA regions, whereby transcription of those specific regions, driven by the inserted promoter, is enhanced significantly compared to endogenous transcription. By growing the mutant pool under different conditions and repeating the sequencing it is possible to observe changes in the number of reads, indicating not only which DNA region contributes to growth and/or survival, but also the relative contribution. The higher levels of specific antibiotic target transcription (driven by the transposon-inserted promoters) will favour bacterial survival and link insertion site to DNA region by proximity.

The position of the inserted promoter can be assessed with respect to its contribution to increased transcription of relevant downstream DNA sequences. A mathematically/technically straightforward bioinformatics component of this technique permits recognition of the contribution of the inserted promoter sequence to transcription of the putative gene. For example, the partial gene transcript may still encode enough information to allow translation of a truncated, but functional protein. Bioinformatics would allow the effects of transcriptional read through on genes downstream of the gene adjacent to the inserted transposon to be considered, where there is there no defined RNA transcription termination sequence.

For example, a transposon/promoter upstream of genes A, B and C may generate a polycistronic transcript of all three genes (A-C), upstream of B a polycistronic transcript of genes B and C and upstream of C just gene C. If the reads for the first two transposons were high and the third low in antibiotic then the antibiotic target would be gene B.

### Biotechnological applications of the engineered mutant bacteria of the invention

These include: bioremediation; microbial enhanced oil recovery; wastewater treatment; sewage treatment; food production; energy production; bio-production; bio-digestion/biodegradation; vaccines; biosensors; probiotics; biocatalysis; biological control; and drug delivery vehicles.

### Exemplification

### Example 1: Production of mutant bacteria which exhibit improved survival and/or growth in the presence of fosfomycin

### (i) Construction of activating transposon (Tn_{A})

Plasmids were constructed which incorporate amplifiable nucleotide sequences which act as transposons. The elements of the transposon include the 19bp mosaic ends which are recognised by a specific transposase enzyme and delimit the transposon, an antibiotic - resistance gene to select for transformants that have resulted from transposition, and an outward oriented promoter at one end of the transposon to activate expression of target genes adjacent to the transposon insertion site .

Alternative plasmids have been constructed with different outward oriented promoters from different genes from *E. coli,* Acinetobacter, Pseudomonas or Rhodopseudomonas. Table 1 provides details of the different promoters used. In addition, different host species bacteria require different antibiotic resistance genes to select for transformants, e.g. chloramphenicol resistance may be used in *E. coli,* kanamycin resistance in *Acinetobacter* spp. and gentamicin resistance in *Pseudomonas* spp. Details of different resistance genes used are given in Table 2.

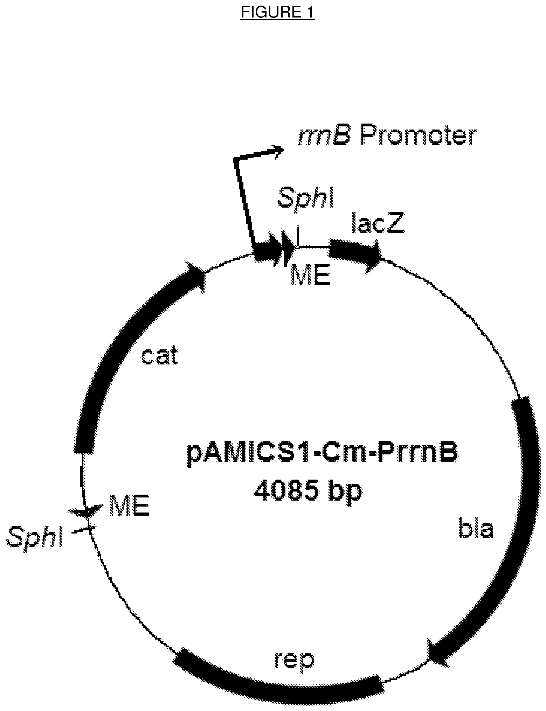

Figure 1 is a genetic map of plasmid pAMICS1-Cm-P*rrnB*. The transposon is flanked by the mosaic ends, ME, and the outward oriented rrnB promoter in indicated. Other components of the plasmid outside of the transposon are from the plasmid vector pBluescript (Agilent), lacZ, beta-galactosidase subunit; bla, beta-lactamase coding for ampicillin resistance; rep, pBR322 replication origin; cat, chloramphenicol acetyltransferase coding for chloramphenicol resistance.

Other suitable plasmid-derivatives are similar to pAMICS1-Cm-P*rrn*B, except that the outward-oriented *rrnB* promoter is substituted for promoters listed in Table 1 and the chloramphenicol resistance determinant is substituted for those listed in Table 2.

**Table 1**

| **Promoter** | **Bacteria** | **Source** | **Accession No.** |
|---|---|---|---|
| tac | *E.coli* | pKK223 | X95387 |
| rplJ | *E.coli* | *E. coli* UTI89 | CP000243 |
| rrnB | *E.coli* | *E. coli* UTI89 | CP000243 |
| rrn | *Acinetobacter baumannii* | ATCC 17978 | CP000521 |
| rplJ | *Acinetobacter baumannii* | ATCC 17978 | CP000521 |
| rrnB | *Pseudomonas aeruginosa* | ATCC 15692 | AE004091 |
| rpsJ | *Pseudomonas aeruginosa* | ATCC 15692 | AE004091 |
| rplK | *Pseudomonas aeruginosa* | ATCC 15692 | AE004091 |
| Weak (respiratory from RP chromosome) | *Rhodopseudomonas palustris* (RP) | NCIMB11774 | BX571963 |
| Medium (ribosomal from RP chromosome) | *Rhodopseudomonas palustris* (RP) | NCIMB11774 | BX571963 |
| Strong (ribosomal from RP chromosome) | *Rhodopseudomonas palustris* (RP) | NCIMB11774 | BX571963 |

**Table2**

| **Resistance gene** | **Source plasmid** | **Source of sequence** | **Used in:** |
|---|---|---|---|
| Kanamycin | Kanamycin GenBlock | | *Acinetobacter baumannii* |
| Chloramphenicol | pACYC184 | X06403.1 | *E. coli* |
| Gentamicin | pFastBad | Invitrogen | *Pseudomonas aeruginosa* and *Rhodopseudomonas palustris* |

### Preparation of transposomes

Transposon DNA fragments for use in transposon mutagenesis were PCR amplified using specific oligos. For generation of the PCR templates, pAMICS1-Cm-P plasmids were digested with the restriction endonuclease Sphl (obtained from New England Biolabs) which cleaves the plasmid at either side of the mosaic ends and the 1.2 kb transposon fragments generated were isolated following electrophoresis through 1% agarose using a "MinElute gel extraction kit" (obtained from QIAGEN). This step prevents the later generation of transformants due to carry-through of plasmid template in the transposon mutagenesis process, ensuring the transformants obtained are transposon mutants and not due to transformants harbouring the plasmid used as the template to generate the transposon fragments.

PCR amplification of the transposon fragments was carried out using "PfuUltra II Fusion" DNA polymerase, which is a "proof-reading" DNA polymerising enzyme (obtained from Agilent). The PCR amplified, de-salted DNA fragment was then treated with polynucleotide kinase to phosphorylate the 5'-DNA ends. Transposomes was then prepared using the purified transposon fragment and recombinant Tn5 transposase.

### Preparation of electrocompetent Escherichia coli

Electrocompetent cells for transposon mutagenesis were prepared by inoculating 5 ml of LB-broth with *Escherichia coli* (ST131) colonies streaked from a frozen stock. This culture was incubated overnight with shaking to promote growth. Typically 2 ml was then used to inoculate 400 ml of pre-warmed 2 x YT broth and the culture incubated at 37°C until the optical density measured at a wavelength of 600 nm was between 0.2 and 0.3. Cells were harvested by centrifugation and washed by resuspending in 10% glycerol. Cells were retrieved by centrifugation and again resuspended in 10% glycerol. This step was repeated until the cells had been washed at least 3 times. Finally, cells were resuspended using a volume of 10% glycerol which is 1/1000 of the volume of the 2 x YT culture (e.g. 400 µl).

### Generation of Escherichia coli Transposon mutant pools

60 µl of the cell suspension described in the previous step were mixed with 0.2 µl of transposomes, and the mixture electroporated using a "Gene II Pulser" (BioRad). Typically, electroporation cuvettes of 2mm electrode gap were used with electroporation settings of 2.5 kV, 25 µF and 200 Ω. These settings resulted in a time constant of 4.8 to 5 msec. 1 ml of S.O.C. broth was then added to the cuvette and, after mixing, the cell suspension transferred to a fresh tube and incubated for 1h 30mins at 37 °C. The cell suspension was then spread on LB-agar plates supplemented with the appropriate concentration of chloramphenicol (e.g. 7.5 µl/ml), and the agar plates were incubated at 37 °C to allow growth of chloramphenicol-resistant transformant colonies resulting from transposition of the transposon into the bacterial genome.

The numbers of colonies obtained were then estimated so as to provide an approximate number of transposon mutants. Colonies were then harvested from the agar plates by resuspending in a volume of LB-broth using a bacteriological spreader. To this cell suspension glycerol was added to a concentration of 15% and the suspension split into aliquots for storage at -80°C.

### Generation of genomic DNA from Escherichia coli Transposon mutant pools to investigate genes involved in chosen growth conditions

This step assays every gene in the bacterial genome for contribution to growth under any chosen condition. It allows the ordering of every gene according to relative contribution to the growth condition, starting with genes that contribute significantly, through genes that provide no significant contribution, through to the genes that are disadvantageous for growth of the bacterial cell under the chosen conditions.

In this example, the selected growth condition was the presence of the antibiotic Fosfomycin.

Pools of the transposon mutant bacteria are grown in the presence of the antibiotic. Typically, this may be performed in 10 ml broth cultures to which 10⁸ individual bacterial transposon mutants have been added from an aliquot of the -80°C frozen stock. Several cultures should be included each with a different concentration of antibiotic. For example, concentrations which are ½ x the minimum inhibitory concentration (MIC), 1 x MIC and 2 x MIC may be performed. Experiments with transposon mutant pools harbouring transposons with the differing promoters (*tac, rplJ* or *rrnB*) may be performed in a single pool, or more cultures may be included if the different mutant pools are to be investigated separately.

Following incubation of the cultures at 37°C overnight in media luria broth or broth supplemented with 60 µM or 150 µM Fosfomycin , 0.1 ml of culture was then transferred to a fresh 10ml LB-broth supplemented with the same concentration of Fosfomycin under investigation, and this culture incubated for at least 6 h to allow growth of the bacteria. Bacteria were then harvested from the culture by centrifugation and genomic DNA extracted using a "Genomic DNA buffer set" and "Tip 100/Gs" (obtainable from QIAGEN). Finally, genomic DNA was dissolved in 10mM Tris (pH8).

### Generation of sequence reads directly from transposon insertions sites using next generation sequencing

Genomic DNA was sheared to create relatively short fragments in a range of approximately 300 - 600 bp, end repaired, 5'-adenylated to allow T-A ligation and ligated to splinkeretted adapters.

To enrich for transposon-border DNA fragments for sequencing, PCR amplifications were then performed using one transposon-specific oligonucleotide and one adapter-specific oligonucleotide that will hybridise to the splinkeretted adapter only after it has been replicated by polymerisation primed from a transposon end. Amplifications were performed in parallel for the left border and right border of the transposon. Two rounds of PCR were run, the second one with a nested primer set to enhance specificity. To suppress amplification derived from splinkerettes, second round PCR included a non-elongatible primer for splinkerettes ("restricted PCR"). Unwanted "activation" of splinkerettes due to a splicing by overlap extension-PCR was further reduced by blocking all DNA ends in splinkerette-libraries by addition of a dideoxynucleotide.

Depending on the transposon used, the binding site for the oligonucleotide primer of this first round of PCR should be 60 to 100 bp away from the transposon mosaic ends, and reactions may be performed using different oligonucleotides specific for "left" and "right" border amplification. First round PCR utilised 100ng template DNA and was run for 14 temperature cycles. Upon completion part of the this resulting reaction was then treated with Exonuclease I to remove first round primers and diluted 1 : 5 with water. 1 µl of the diluted material was used as template in the second round (20 µl) PCR, which was run for 18 temperature cycles. One of the nested oligonucleotide primers used for the second round PCR should hybridise to the transposon ends. Both of the oligonucleotides used in the second round PCR possessed random sequence of five nucleotides at the 5'end, which assisted with cluster registration when sequencing was performed on the Illumina HiSEQ sequencing machine.

Sequencing adapters were then ligated to the DNA fragment libraries and the DNA prepared for sequencing using the standard Illumina sequencing protocol for 100 bp paired end sequencing.

### Mapping of sequence reads to a reference genome

The process of reading records from the two Illumina sequencer fastq files was synchronised, so that the program could check for the presence of one transposon sequence and one splinkerette sequence in each read pair. The transposon and splinkerette sequences were clipped from the front of each read, and only read pairs which had a transposon in one read and a splinkerette in the other assigned as valid. All other sequence pairs were removed as the records were processed. A single output file was then produced, in fastq format, containing clipped transposon reads only.

The reads from this fastq file were mapped to a reference genome to create a .mapview file which contained the genomic position that the reads mapped to, and whether they mapped to the forward or reverse strand. This gave important information about the direction of the transposon insertion.

The program tpnlnsertionSites.pl (Sanger Institute) read the .mapview file and produced three output files containing insert and read data for both forward and reverse strands, based on the mapping data in the .mapview file, and a separate file containing the combined insert and read data for both strands. The program normGeneCombinedFreq.pl read the data from the forward, reverse and combined insert site files, and calculated the number of inserts and read upstream of each gene, allowing for the direction of the transposon's insertion. The program also calculated the insert and read counts within genes, and output this to a separate file. The data was analysed using ARTEMIS (Sanger Institute).

### Identification of genes advantageous for growth in the presence of fosfomycin

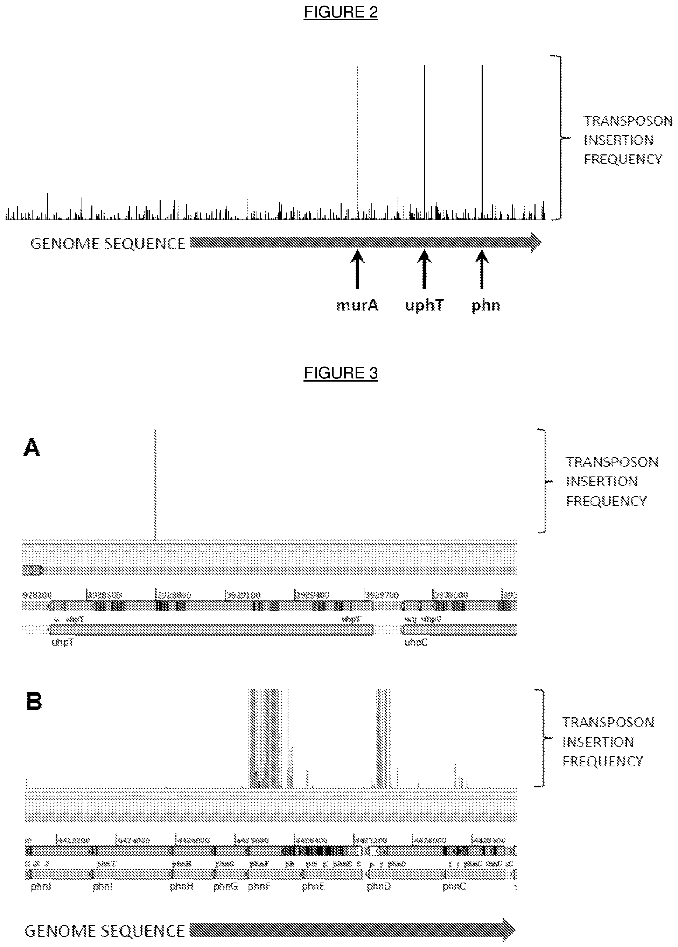

Mapping of sequence reads to an *E. coli* reference genome indicated 3 major loci to which mapped very large numbers of reads, indicating the survival of significant numbers of mutants with transposon insertions at these loci and therefore that these loci are important for the survival of mutants in the presence of Fosfomycin. Figure 2 shows the whole genome sequence of *E*. *coli* ST131 showing insertion points and frequency of activator transposon insertion. These loci were *murA, phn* operon and *uhpT.*

Figure 3 shows insertion maps for *phn* and *uhpT*: the specific genome region of *E. coli* ST131 is shown, together with gene insertion points and frequency of activator transposon insertion for *uhpT* (panel A) and the *phn* operon (panel B).

The *murA* gene codes for UDP-N-acetylglucosamine-1-carboxyvinyltransferase, an essential gene required for biosynthesis of the bacterial murein sacculus (peptidoglycan layer) and the known target of Fosfomycin action. Sequence reads generated from transposon insertions mapped immediately upstream of *murA* resulting in disruption of the *yrbA* gene (also known as *ibaG* which is involved in the acid tolerance response).

The *phn* operon encodes the carbon-phosphorus lyase complex. Very large numbers of reads mapped to the *phnF*gene which codes for the phosphonate metabolism transcriptional regulator. Insertions in this gene were also immediately upstream of the *phnG* gene and other genes within the same operon, (*phnA-P*), which codes for the carbon-phosphorus lyase complex. This complex breaks carbon-phosphorus bonds, the like of which are present in Fosfomycin, and the breaking of which result in inactivation of Fosfomycin. Large numbers of reads also mapped to the 3'-half of the *phnD* gene coding for a phosphonate ABC-transporter substrate-binding protein, and immediately upstream of the *phnE* gene coding for a phosphonate ABC transporter permease protein.

The *uhpT* gene codes for the hexose phosphate transporter a gene product mainly responsible for transporting Fosfomycin into the bacterial cell. There was a major insertion point within this gene where a large number of transposons were observed, which would have caused gene disruption, preventing the production of a viable transporter protein.

### Example 2: Production of mutant Rhodopseudomonas palustris exhibiting improved growth in industrial waste

*R. palustris* is a purple photosynthetic bacterium that belongs to the alpha proteobacteria. It has extraordinary metabolic versatility, growing by any one of the four modes of metabolism that support life: photoautotrophic or photosynthetic (energy from light and carbon from carbon dioxide), photoheterotrophic (energy from light and carbon from organic compounds), chemoheterotrophic (carbon and energy from organic compounds) and chemoautotrophic (energy from inorganic compounds and carbon from carbon dioxide). This metabolic flexibility facilitates the use of this bacterium in biotechnological applications, including bioremediation of industrial wastes.

### Preparation of electrocompetent Rhodopseudomonas palustris

Electrocompetent cells for transposon mutagenesis were prepared by inoculating 500 ml of Yeast Peptone Dextrose (YPD) broth with *Rhodopseudomonas palustris* colonies streaked from a frozen stock. This culture was incubated overnight at 30°C in the presence of light with gentle shaking to ensure that the culture remained dispersed. 1 litre of YPD was inoculated with 10 ml of the overnight culture, and incubated at 30°C until the optical density measured at a wavelength of 600 nm 0.3. Cells were harvested by centrifugation and re-suspended in 10% glycerol. Cells were retrieved by centrifugation and again re-suspended in 10% glycerol. This step was repeated until the cells had been washed five times in total. Cells were re-suspended using a volume of glycerol that is 100th the volume of the starting culture.

### Generation of Rhodopseudomonas Palustris transposon mutant pools

50 µl of the electrocompetent cells were mixed with 1 µl of transposome, then electroporated using a 'Gene II pulser" (Biorad). Routinely, electroporation cuvettes with a 2 mm electrode gap were used with electroporation settings of 2.0 kV, 25 µF and 200 Ω. 1 ml of YPD was then added to the cuvette and after mixing, the resultant cell suspension was transferred to a fresh tube and incubated for 1 h at 30 °C. The cell suspension was then spread onto YPD agar plates, containing the appropriate concentration of gentamicin, and the agar plates were incubated at 30 °C to allow growth of gentamicin resistant colonies, resulting from transposition of the transposon into the bacterial genomes. Colonies were then harvested from the agar plates by re-suspending in YPD broth. Glycerol was then added to a concentration of 15% and the suspension split into aliquots for storage at -80 °C.

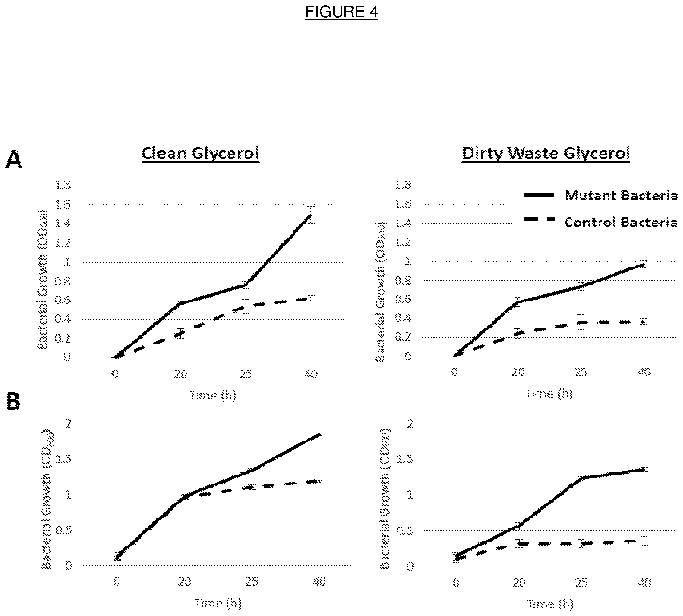

*Rhodopseudomonas palustris* transposon mutant pools using the three promoters shown in table 1 were generated to investigate growth in industrial waste (contaminated glycerol), both in the presence and absence of light (panels A and B, respectively). As shown in Figure 4, *R. palustris* mutants were generated which exhibited improved growth in both clean and dirty glycerol, under both chemotrophic and phototrophic modes of metabolism. These data show that mutant forms of *R. palustris* which are significantly better adapted to growth in industrial waste (dirty glycerol) may be readily engineered and selected by the processes of the invention.

## Claims

1. A process for producing a mutant bacterium which exhibits improved survival and/or growth under a selected growth condition, the process comprising the steps of:
(a) generating a pool of mutant bacteria by transposon mutagenesis with two or more different activating transposons (Tn_{A}s), wherein each Tn_{A} comprises a promoter such that transposon insertion into bacterial DNA disrupts the function or increases the transcription of a gene at or near the insertion site in a position-dependent manner, and wherein the transposon mutagenesis yields an insertion rate of at least one transposon per 10 base pairs of bacterial DNA;
(b) growing bacteria from the mutant pool under the selected growth condition and under one or more reference conditions to produce two or more test cultures;
(c) comparing the distribution of Tn_{A} insertions between test cultures to identify a first class of genes which are disadvantageous for growth and/or survival under the selected growth condition and a second class of genes which are advantageous for growth and/or survival under the selected growth condition; and
(d) providing an engineered mutant bacterium in which at least one of said disadvantageous genes is removed or disrupted and/or at least one of said advantageous gene is overexpressed, such that the mutant bacterium exhibits improved survival and/or growth under the selected growth condition.

2. The process of claim 1 wherein a plurality of said disadvantageous genes is removed or disrupted and/or a plurality of said advantageous genes is overexpressed.

3. The process of claim 1 or claim 2 further comprising culturing the engineered mutant bacterium and then applying steps (a)-(c) of claim 1 to said engineered mutant bacterium to identify a further first class of genes which are disadvantageous for growth and/or survival under the selected growth condition and a further second class of genes which are advantageous for growth and/or survival under the selected growth condition, optionally further comprising the step of providing a second round engineered mutant bacterium in which at least one gene of said further first class of disadvantageous genes is removed or disrupted and/or at least one gene of said further second class of advantageous gene is overexpressed, such that the mutant bacterium exhibits improved survival and/or growth under the selected growth condition relative to the engineered mutant bacterium of claim 1.

4. The process of claim 3 comprising one or more further rounds of mutagenesis and iterative application of steps (a) to (c) of claim 1 to provide a third or greater round mutant bacterium which exhibits improved survival and/or growth in the presence of said environmental challenge relative to the engineered mutant bacterium of the previous round.

5. The process of any one of the preceding claims wherein the removal and/or disruption of said disadvantageous genes comprises genome minimization.

6. The process of claim 5 wherein said genome minimization comprises integration of plasmid DNA into the bacterial chromosome and subsequent resolution of the cointegrate.

7. The process of claim 5 wherein said genome minimization comprises homologous recombination mediated by short homology arms at the ends of a linear DNA.

8. The process of any one of the preceding claims further comprising the step of introducing at least one heterologous gene into the bacterium, optionally wherein said heterologous gene is advantageous for growth and/or survival under the selected growth condition, for example wherein said heterologous gene encodes a therapeutic protein.

9. The process of claim 8 comprising the step of introducing a heterologous gene cluster into the bacterium, optionally wherein the heterologous gene cluster encodes a biosynthetic or biodegradative pathway.

10. The process of any of the preceding claims wherein the selected growth condition comprises the presence of:
(a) an environmental contaminant;
(b) an industrial waste product;
(c) a medical waste product;
(d) a drug or candidate drug;
(e) a selected carbon source, for example a hydrocarbon; or
(f) one or more other organisms, for example microorganisms and viruses.

11. The process of claim 10 wherein said one or more other organisms are selected from:
(a) human pathogens;
(b) animal pathogens; and
(c) plant pathogens.

12. The process of any one of the preceding claims wherein the pool of mutant bacteria comprises: (a) at least 0.5 x 10⁵ mutants, for example at least 1 x 10⁵ mutants; (b) at least 5x10⁵ mutants; (c) at least 1x10⁶ mutants; (d) 0.5 x10⁶ to 2 x10⁶ mutants; or (e) about 1 x10⁶ mutants.

13. The process of any one of the preceding claims wherein the bacterial DNA of step (a) is selected from chromosomal (genomic) DNA, plasmid DNA or a mixture of chromosomal (genomic) and plasmid DNA.

14. The process of any one of the preceding claims wherein the bacterium is a Gram-positive or Gram-negative bacterium, for example *Rhodopseudomonas palustris.*

15. The process of any one of the preceding claims wherein the distribution of Tn_{A} insertions between test cultures is compared by sequencing DNA adjacent or near the insertion site of the Tn_{A}.

## Patentansprüche

1. Verfahren zum Erzeugen eines mutanten Bakteriums, das verbessertes Überleben und/oder Wachstum unter ausgewählten Wachstumsbedingungen aufweist, wobei das Verfahren die folgenden Schritte umfasst:
(a) Erzeugen eines Pools mutanter Bakterien durch Transposon-Mutagene mit zwei oder mehr unterschiedlichen aktivierender Transposons (Tn_{A}s), wobei jedes Tn_{A}s einen Promotor umfasst, so dass die Transposon-Insertion in Bakterien-DNA die Funktion beeinträchtigt oder die Transkription eines Gens an oder nahe an dem Insertionsort positionsabhängig erhöht, und wobei die Transposon-Mutagenese eine Insertionsrate von mindestens einem Transposon je 10 Basenpaaren Bakterien-DNA ergibt;
(b) Züchten von Bakterien aus dem mutanten Pool unter ausgewählten Wachstumsbedingungen und unter einer oder mehreren Referenzbedingungen, um zwei oder mehr Testkulturen zu erzeugen;
(c) Vergleichen der Verteilung der Tn_{A}-Insertionen zwischen Testkulturen, um eine erste Klasse von Genen zu identifizieren, die für das Wachstum und/oder Überleben unter den ausgewählten Wachstumsbedingungen nachteilig sind, und eine zweite Klasse von Genen, die für das Wachstum und/oder Überleben unter den ausgewählten Wachstumsbedingungen vorteilhaft sind; und
(d) Bereitstellen eines veränderten mutanten Bakteriums, in dem mindestens eines der nachteiligen Gene entfernt oder gestört ist und/oder mindestens eines der vorteilhaften Gene überexprimiert ist, so dass das mutante Bakterium verbessertes Überleben und/oder Wachstum unter ausgewählten Wachstumsbedingungen aufweist.

2. Verfahren nach Anspruch 1, wobei eine Mehrzahl der nachteiligen Gene entfernt oder gestört wird, und/oder wobei eine Mehrzahl der vorteilhaften Gene überexprimiert wird.

3. Verfahren nach Anspruch 1 oder 2, ferner umfassend das Züchten des veränderten mutanten Bakteriums und das folgende Anwenden der Schritte (a) bis (c) aus Anspruch 1 auf das veränderte mutante Bakterium, um eine weitere erste Klasse von Genen zu identifizieren, die für das Wachstum und/oder Überleben unter den ausgewählten Wachstumsbedingungen nachteilig sind, und eine weitere zweite Klasse von Genen, die für das Wachstum und7oder Überleben unter den ausgewählten Wachstumsbedingungen vorteilhaft sind, optional ferner den Schritt des Bereitstellens eines in zweiter Runde veränderten mutanten Bakteriums umfassend, wobei mindestens ein Gen der weiteren ersten Klasse nachteiliger Gene entfernt oder gestört ist, und/oder wobei wenigstens ein Gen der weiteren zweiten Klasse vorteilhafter Gene überexprimiert ist, so dass das mutante Bakterium unter den ausgewählten Wachstumsbedingungen im Vergleich zu dem veränderten mutanten Bakterium aus Anspruch 1 verbessertes Überleben und/oder Wachstum aufweist.

4. Verfahren nach Anspruch 3, eine oder mehrere weitere Runden der Mutagenese und iterativen Anwendung der Schritte (a) bis (c) aus Anspruch 1 umfassend, um ein mutantes Bakterium der dritten oder höherer Runde bereitzustellen, das verbessertes Überleben und/oder Wachstum in Gegenwart der Umgebungsherausforderung im Verhältnis zu dem veränderten mutanten Bakterium der vorherigen Runde aufweist.

5. Verfahren nach einem der vorstehenden Ansprüche, wobei das Entfernen und/oder Stören der nachteiligen Gene Genomminimierung umfasst.

6. Verfahren nach Anspruch 5, wobei die Genomminimierung die Integration von Plasmid-DNA in das Bakterienchromosom und folgende Resolution des Cointegrats umfasst.

7. Verfahren nach Anspruch 5, wobei die Genomminimierung homologe Rekombination umfasst, vermittelt durch kurze Homologiearme an den Enden einer linearen DNA.

8. Verfahren nach einem der vorstehenden Ansprüche, ferner umfassend den Schritt der Insertion wenigstens eines heterologen Gens in das Bakterium, wobei das heterologe Gen optional vorteilhaft ist für Wachstum und/oder Überleben unter ausgewählten Wachstumsbedingungen, wobei das heterologe Gen etwa für ein therapeutisches Protein codiert.

9. Verfahren nach Anspruch 8, das den Schritt der Insertion eines heterologen Gen-Clusters in das Bakterium umfasst, wobei das heterologe Gen-Cluster optional für einen biosynthetischen oder biologisch abbauenden Pfad codiert.

10. Verfahren nach einem der vorstehenden Ansprüche, wobei die ausgewählten Wachstumsbedingungen folgende Präsenz umfassen:
(a) einer die Umgebung kontaminierenden Substanz;
(b) eines industriellen Abfallprodukts;
(c) eines medizinischen Abfallprodukts;
(d) eines Arzneimittels oder Arzneimittelkandidaten;
(e) einer ausgewählten Kohlenstoffquelle, wie etwa Kohlenwasserstoff; oder
(f) eines oder mehrerer sonstiger Organismen, wie etwa Mikroorganismen oder Viren.

11. Verfahren nach Anspruch 10, wobei der eine oder die mehreren sonstigen Organismen ausgewählt sind aus:
(a) menschlichen Pathogenen;
(b) tierischen Pathogenen; und
(c) pflanzlichen Pathogenen.

12. Verfahren nach einem der vorstehenden Ansprüche, wobei der Pool mutanter Bakterien folgendes umfasst: (a) mindestens 0,5 x 10⁵ Mutanten, z.B. mindestens 1 x 10⁵ Mutanten; (b) mindestens 5 x 10⁵ Mutanten; (c) mindestens 1 x 10⁶ Mutanten; (d) 0,5 x 10⁶ bis 2 x 106 Mutanten; oder (e) etwa 1 x 10⁶ Mutanten.

13. Verfahren nach einem der vorstehenden Ansprüche, wobei die Bakterien-DNA aus Schritt (a) ausgewählt ist aus chromosomaler (genomer) DNA, Plasmid-DNA oder einer Mischung aus chromosomaler (genomer) und Plasmid-DNA.

14. Verfahren nach einem der vorstehenden Ansprüche, wobei das Bakterium ein grampositives oder gramnegatives Bakterium ist, zum Beispiel *Rhodopseudomonas palustris.*

15. Verfahren nach einem der vorstehenden Ansprüche, wobei die Verteilung von Tn_{A}-Insertionen zwischen Testkulturen durch DNA-Sequenzierung angrenzend an oder nahe des Insertionsorts des Tn_{A} verglichen wird.

## Revendications

1. Procédé de production d'une bactérie mutante qui présente une survie et/ou croissance améliorées dans une condition de culture sélectionnée, le procédé comprenant les étapes consistant à :
(a) générer un pool de bactéries mutantes par mutagenèse par transposon avec au moins deux transposons d'activation (Tn_{A}) différents, chaque Tn_{A} comprenant un promoteur tel que l'insertion de transposon dans l'ADN bactérien disrupte la fonction ou augmente la transcription d'un gène au niveau ou à proximité du site d'insertion d'une manière dépendant de la position, et la mutagenèse par transposon produisant un taux d'insertion d'au moins un transposon par 10 paires de bases d'ADN bactérien ;
(b) cultiver des bactéries du pool de mutants dans les conditions de croissance sélectionnées et dans au moins une condition de référence pour produire au moins deux cultures d'essai ;
(c) comparer la distribution des insertions de Tn_{A} entre les cultures d'essai pour identifier une première classe de gènes qui sont désavantageux pour la croissance et/ou la survie dans la condition de croissance sélectionnée et une seconde classe de gènes qui sont avantageux pour la croissance et/ou la survie dans la condition de croissance sélectionnée ; et
(d) fournir une bactérie mutante manipulée dans laquelle au moins l'un desdits gènes désavantageux est éliminé ou disrupté et/ou au moins l'un desdits gènes avantageux est surexprimé, de sorte que la bactérie mutante présente une survie et/ou une croissance améliorée dans la condition de croissance sélectionnée.

2. Procédé selon la revendication 1, une pluralité desdits gènes désavantagés étant retirée ou disruptée et/ou une pluralité desdits gènes avantageux étant surexprimée.

3. Procédé selon la revendication 1 ou 2, comprenant en outre les étapes consistant à cultiver la bactérie mutante manipulée puis à appliquer les étapes (a) à (c) de la revendication 1 à ladite bactérie mutante manipulée pour identifier une première classe supplémentaire de gènes qui sont désavantageux pour la croissance et/ou la survie dans la condition de croissance sélectionnée et une seconde classe supplémentaire de gènes qui sont avantageux pour la croissance et/ou la survie dans la condition de croissance sélectionnée, comprenant en outre éventuellement l'étape consistant à fournir une bactérie mutante manipulée de second cycle dans laquelle au moins un gène de ladite première classe supplémentaire de gènes désavantageux est retiré ou disrupté et/ou au moins un gène de ladite seconde classe supplémentaire de gènes avantageux est surexprimé, de sorte que la bactérie mutante présente une survie et/ou croissance améliorée dans la condition de croissance sélectionnée par rapport à la bactérie mutante manipulée selon la revendication 1.

4. Procédé selon la revendication 3 comprenant un ou plusieurs autres cycles de mutagenèse et d'application itérative des étapes (a) à (c) de la revendication 1 pour fournir une bactérie mutante de troisième cycle ou plus qui présente une survie et/ou une croissance améliorée en présence dudit défi environnemental par rapport à la bactérie mutante modifiée du cycle précédent.

5. Procédé selon l'une quelconque des revendications précédentes, l'élimination et/ou la disruption desdits gènes désavantageux comprenant la minimisation du génome.

6. Procédé selon la revendication 5, ladite minimisation du génome comprenant l'intégration de l'ADN plasmidique dans le chromosome bactérien et la résolution subséquente du coïntégrat.

7. Procédé selon la revendication 5, ladite minimisation du génome comprenant la recombinaison homologue médiée par des bras homologiques courts aux extrémités d'un ADN linéaire.

8. Procédé selon l'une quelconque des revendications précédentes, comprenant en outre l'étape consistant à introduire au moins un gène hétérologue dans la bactérie, éventuellement ledit gène hétérologue étant avantageux pour la croissance et/ou la survie dans la condition de croissance sélectionnée, par exemple ledit gène hétérologue codant une protéine thérapeutique.

9. Procédé selon la revendication 8 comprenant l'étape consistant à introduire une famille de gènes hétérologues dans la bactérie, éventuellement la famille de gènes hétérologues codant une voie biosynthétique ou biodégradable.

10. Procédé selon l'une quelconque des revendications précédentes, la condition de croissance sélectionnée comprenant la présence de :
(a) un contaminant de l'environnement ;
(b) un déchet industriel ;
(c) un déchet médical ;
(d) un médicament ou un médicament candidat ;
(e) une source de carbone sélectionnée, par exemple un hydrocarbure ; ou
(f) au moins un autre organisme, par exemple des micro-organismes et des virus.

11. Procédé selon la revendication 10, ledit au moins un organisme étant choisi parmi :
(a) les agents pathogènes humains ;
(b) les agents pathogènes animaux ; et
(c) les agents pathogènes végétaux.

12. Procédé selon l'une quelconque des revendications précédentes, le pool de bactéries mutantes comprenant : (a) au moins 0,5 x 10⁵ mutants, par exemple au moins 1 x 10⁵ mutants ; (b) au moins 5x10⁵ mutants ; (c) au moins 1 x10⁶ mutants ; (d) 0,5 x10⁶ à 2 x10⁶ mutants ; ou (e) environ 1 x10⁶ mutants.

13. Procédé selon l'une quelconque des revendications précédentes, l'ADN bactérien de l'étape (a) étant choisi parmi l'ADN chromosomique (génomique), l'ADN plasmidique ou un mélange d'ADN chromosomique (génomique) et d'ADN plasmidique.

14. Procédé selon l'une quelconque des revendications précédentes, la bactérie étant une bactérie Gram-positive ou Gram-négative, par exemple *Rhodopseudomonas palustris.*

15. Procédé selon l'une quelconque des revendications précédentes, la distribution des insertions de Tn_{A} entre les cultures d'essai étant comparée par séquençage de l'ADN adjacent ou proche du site d'insertion du Tn_{A}.
